# EUROPEAN PATENT APPLICATION

(11) **EP 2 868 264 A2**
(43) Date of publication of application: **06.05.2015**
(21) Application number: 14188887.5
(22) Date of filing: 14.10.2014
(51) Int. Cl.: A61B 5/00, A61B 5/04

(54) **System and method for acquisition of biopotential signals with electrode-tissue impedance measurement.**

(30) Priority: 31.10.2013 EP 13191015
(71) Applicant: IMEC, 3001 Leuven (BE)
(72) Inventor: Torfs, Tom, 3001 Leuven (BE); Yazicioglu, Refet Firat, 3001 Leuven (BE)
(74) Representative: Clerix, André

(57) **Abstract**

The invention relates to a system for acquisition of biopotential signals, comprising at least a first electrode (E1) configured for detecting a biopotential signal within a signal bandwidth of interest and being connected to an impedance detection module (100) which provides a first electrode voltage (VO1); said impedance detection module (100) comprising a current generation circuit (40) connected in parallel to an amplifier (A1), the current generation circuit (40) comprising an AC current generator (AC1) configured to generate a first current signal (IS1) through said first electrode, said first current signal (IS1) having a frequency outside of the signal bandwidth of interest; and a capacitor (CS1) connected between the input of the amplifier (A1) and the AC current generator (AC1) so as to isolate said AC current generator from said amplifier input at the signal bandwidth of interest; and signal processing means (300) for calculating a component value (IMP) of a first and a second electrode-tissue impedance (Z1, Z2, ZB) based on the difference between the first electrode voltage (VO1) and a second electrode voltage (VO2 to VON; BV).

## Description

### Technical Field

The present invention relates generally to the field of biopotential signal acquisition systems and more specifically to a system and a method in the art with electrode-tissue impedance measurement capabilities.

### Background

Ambulatory monitoring of biopotential signals (ECG, EEG, EMG, etc.) is a highly relevant topic in personal healthcare. A key technical challenge in such application environments is overcoming motion artifacts that significantly affect the recorded biopotential signals. A possible approach to tackle this problem is to collect data from other sensors that have maximum correlation with the motion artifact signal and minimal correlation with the biopotential signals. Some known systems measure the electrode-tissue impedance, which is then used as a reference signal input for removing such motion artifacts in the biopotential signal signal.

One known electrode-skin impedance monitoring system is disclosed in article *"*A 2.4µA continuous-time electrode-skin impedance measurement circuit for motion artifact monitoring in ECG acquisition systems", by Sunyoung Kim et al., VLSI Circuits (VLSIC), 2010, pp.219-220, 16-18 June 2010. The electrode-tissue impedance is measured by applying an alternating current (AC) of constant amplitude and frequency into the electrodes and detecting the resulting differential voltage between the electrodes, through the voltage amplifier that also measures the ECG signal.

Another known technique for measuring the electrode-tissue impedance is disclosed in article *"*Correlation Between Electrode-Tissue Impedance and Motion Artifact in Biopotential Recordings", by Dilpreet Buxi et al., IEEE Sensors Journal, Vol. 12, no. 12, December 2012, pp. 3373-3383. In this system the phase of a single current source can be selected between 0° and 180° mode D and mode T respectively. The mode D leads to the generation of a common-mode stimulation current that can be used to monitor the impedance difference between two lead electrodes. And the mode T leads to the generation of a differential stimulation current that can be used to measure the sum of impedances of the lead electrodes or the total impedance.

However, state of the art systems are not well suited for biopotential signal acquisition systems in ambulatory environments where the sensors/electrodes are less tightly strapped to the body and/or no gel is used and therefore some degree of relative sensor to body motion will occur.

### Summary

According to one aspect of the present disclosure, a new system and method for acquisition of biopotential signals is provided.

According to an exemplary embodiment of the present description there is provided system for acquisition of biopotential signals comprising: a first electrode configured for detecting a biopotential signal within a signal bandwidth of interest and being connected to an impedance detection module which provides a first electrode voltage; said impedance detection module comprising a current generation circuit with an AC current generator configured to generate a first current signal through said first electrode, said first current signal having a frequency outside of the signal bandwidth of interest; and an amplifier connected in parallel to said current generation circuit; and signal processing means for calculating a component value of at least a first electrode-tissue impedance based on the difference between the first electrode voltage and a second electrode voltage; wherein the current generation circuit comprises a capacitor connected between the input of the amplifier and the AC current generator so as to isolate said AC current generator from said amplifier input at the signal bandwidth of interest.

According to an exemplary embodiment, the first electrode impedance is greater than 1 megohm.

According to another exemplary embodiment, the signal bandwidth of interest is below 250 hertz.

According to another exemplary embodiment, the first current signal has a frequency greater than 1 kilohertz.

According to another exemplary embodiment, the value of the current generation circuit capacitor is designed such as to reduce the amplifier's input impedance by less than 25%.

According to another exemplary embodiment, the value of the current generation circuit capacitor is designed such as to obtain values of the current generation circuit impedance greater than 10 gigaohm at the signal bandwidth of interest.

According to another exemplary embodiment, the value of the current generation circuit capacitor is between 0.1 to 20 picofarad.

According to another exemplary embodiment, the AC current generator is designed so as to have an output impedance which is at least 5 times higher than the impedance of the capacitor at the frequency of the first current signal.

According to another exemplary embodiment, the system for acquisition of biopotential signals further comprises: a second electrode configured for detecting a biopotential signal within a signal bandwidth of interest and being connected to an impedance detection module which provides a second electrode voltage; said impedance detection module comprising a current generation circuit with an AC current generator configured to generate a second current signal through said second electrode; and wherein the first current signal (IS1) and the second current signal are 180 degrees out of phase.

According to another exemplary embodiment, the system for acquisition of biopotential signals further comprises: a second electrode configured for detecting a biopotential signal within a signal bandwidth of interest and being connected to an impedance detection module which provides a second electrode voltage; said impedance detection module comprising a current generation circuit with an AC current generator configured to generate a second current signal through said second electrode; a bias electrode configured for biasing the subjects body; and so arranged that when the first current signal and the second current signal are in phase, the net resulting current flows into the bias electrode.

According to another exemplary embodiment, at least one of the electrodes is a non-contact or a dry contact electrode. The non-contact electrode may be a non-contact capacitive electrode.

According to an exemplary embodiment the biopotential signal is an ECG, an EEG or an EMG biopotential signal.

According to another aspect of the present description, there is provided a method for acquisition of biopotential signals comprising: detecting a biopotential signal within a signal bandwidth of interest with a first electrode; generating a first current signal through said first electrode, said first current signal having a frequency outside of the signal bandwidth of interest; isolating said first current signal from said detected biopotential signal at the signal bandwidth of interest; generating a first and a second electrode voltage; and calculating a component value of at least a first electrode-tissue impedance based on the difference between the first electrode voltage and a second electrode voltage.

Certain objects and advantages of various new and inventive aspects have been described above. It is to be understood that not necessarily all such objects or advantages may be achieved in accordance with any particular embodiment of the present invention. Those skilled in the art will recognize that the solution of the present invention may be embodied or carried out in a manner that achieves or optimizes one advantage or group of advantages without necessarily achieving other objects or advantages.

### Brief description of the drawings

The above and other aspects of the system and method for acquisition of biopotential signals according to the present invention will be shown and explained with reference to the non-restrictive example embodiment(s) described hereinafter.
**Figures 1A** **and** **1B** show a first exemplary block diagram of a system for acquisition of biopotential signals according to an embodiment of the invention.
**Figures 2A** **and** **2B** show a second exemplary block diagram of a system for acquisition of biopotential signals according to an embodiment of the invention.
**Figure 3** shows a third exemplary block diagram of a system for acquisition of biopotential signals according to an embodiment of the invention.
**Figure 4** shows a fourth exemplary block diagram of a system for acquisition of biopotential signals according to an embodiment of the invention.
**Figures 5A** **and** **5B** show a fifth exemplary block diagram of a system for acquisition of biopotential signals according to an embodiment of the invention.
**Figure 6** shows a sixth exemplary block diagram of a system for acquisition of biopotential signals according to an embodiment of the invention.

### Detailed description

In the following, in the description of exemplary embodiments, various features may be grouped together in a single embodiment, figure, or description thereof for the purpose of streamlining the disclosure and aiding in the understanding of one or more of the various inventive aspects. This is however not to be interpreted as the invention requiring more features than the ones expressly recited in the main claim. Furthermore, combinations of features of different embodiments are meant to be within the scope of the invention, as would be clearly understood by those skilled in the art. Additionally, in other instances, well-known methods, structures and techniques have not been shown in detail in order not to obscure an understanding of the description.

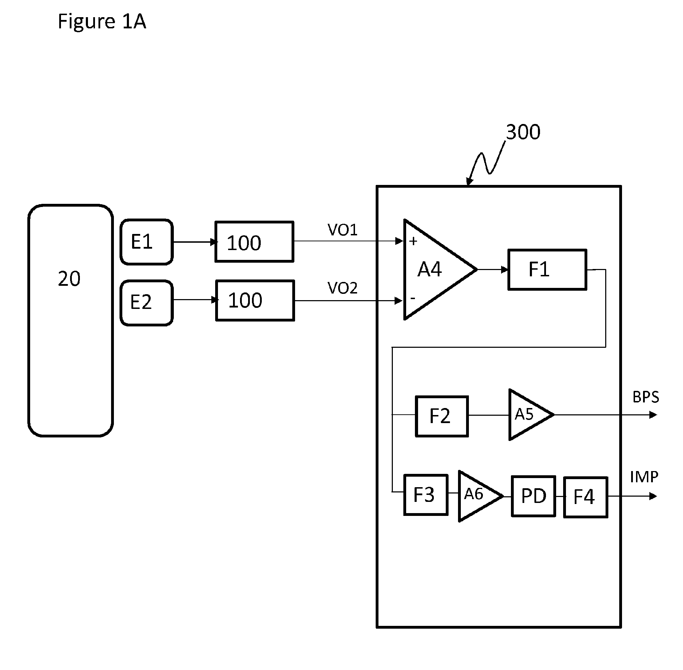

**Figure 1A** shows a first exemplary block diagram of a system for acquisition of biopotential signals according to an embodiment of the invention. The figure illustrates two electrodes **E1, E2** which are applied to a human body **20,** for example attached to or placed in close proximity to the subject's skin or tissue. Each electrode is connected to an impedance detection module **100** which provides an electrode voltage signal **VO1, VO2.** The figure also illustrates schematically a signal processing unit **300,** which basically extracts or calculates, from the first and second voltage electrode signals **VO1, VO2,** a biopotential signal **BPS** and an electrode-tissue impedance signal **IMP.** The signal processing unit **300** may comprise a differential amplifier **A4** and further signal processing means, which extract the biopotential and electrode-tissue impedance signals, which are multiplexed on the same signal but at different frequencies at the output of the differential amplifier **A4.** Said signal processing means may comprise, for example, an optional mains notch **F1,** and in the biopotential signal path, a low-pass filter **F2** and a gain amplifier **A5,** and in the electrode-tissue impedance signal path, a band-pass filter **F3,** a gain amplifier **A6,** a peak detector **PD** and a low-pass filter **F4.**

It is understood that the system may comprise further signal processing means or digital processing units or circuits which may use the electrode-tissue impedance signal **IMP** for motion artifact reduction purposes, for detecting whether and how well an electrode is making contact to the body **20** and/or assessing the quality and variation over time of the electrode connection and, for example, assigning a suitable confidence level to the obtained signals or features extracted out of them or discarding fragments of signals entirely.

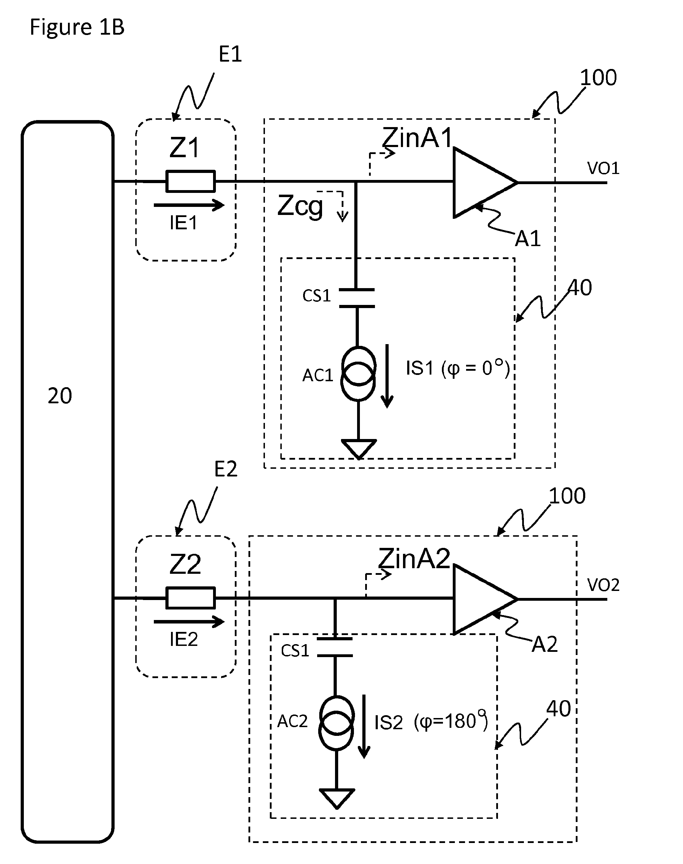

**Figure 1B** shows a more detailed representation of some aspects of the system of **Figure 1A****,** and more specifically, the electrodes **E1, E2** and the impedance detection module **100.** The electrodes **E1, E2** are represented by an electrode-tissue impedance **Z1, Z2** which is connected to a current generation circuit **40** and to an amplifier **A1, A2.** The current generation circuit **40** comprises an AC current generator **AC1, AC2** and a capacitor **CS1** connected in series. Each AC current generator **AC1, AC2** generates a current signal **IS1, IS2, IE1, IE2** through the electrodes which has a frequency outside of the signal bandwidth of interest of the biopotential signal. According to an exemplary embodiment, the frequency of the generated current signal **IS1, IS2** is higher than 1 kHz.

According to an exemplary embodiment of the invention the amplifier **A1** is a high input impedance amplifier. The input impedance **Zcg** of the current generation circuit **40** is also high compared to the input impedance of the biopotential amplifier **A1** so that the total input impedance **ZinA1, ZinA2** is not severely degraded. According to an exemplary embodiment the AC current source **AC1, AC2** with the capacitor **CS1** in series allows maintaining a very large input impedance at signal frequencies of the biopotential signal of interest. According to an exemplary embodiment, the biopotential signal frequencies of interest are below 100 or 250 Hz. According to another exemplary embodiment, the value of the capacitor **CS1** lies in the picofarad range, for example, 1 pF. The AC current source may be designed with relaxed output impedance requirements, for example an output impedance below 1 GΩ.

Also according to an exemplary embodiment of the invention, the capacitor **CS1** advantageously separates or isolates the current source **IS1, IS2** from the biopotential signal at the input of the amplifier **A1, A2.** According to an exemplary embodiment, the series capacitor effectively isolates the current source at biopotential signal frequencies, resulting in an additional input load impedance of 16 GΩ, which will reduce the total input impedance **ZinA1, ZinA2** by only 25%.

Further, according to an exemplary embodiment, the AC current source **AC1, AC2** has an output impedance that is significantly higher than the series impedance of the coupling capacitor **CS1** but only at the frequency of the generated current **IS1, IS2.** For example, in case the frequency of the generated current is 10 kHz, an AC current source **AC1, AC2** with an output impedance higher than 16 MΩ suffices to function as a good AC current source. According to an exemplary embodiment, the AC current source **AC1, AC2** is implemented as a Howland current pump. A Howland current pump can advantageously produce a voltage-controlled output current, simplifying the generation of sinusoidal or arbitrary current shapes, for example through a DAC. Additionally, a single stage can both source and sink current as required. For small currents, e.g. in nA range, a high output impedance can be achieved without high-precision or trimmed resistors.

According to an embodiment of the invention, the impedance detection module **100** applies an AC current **IS1, IS2** of constant amplitude and frequency into the electrodes **E1, E2** and the resulting differential voltage between the electrodes **VO1, VO2** is measured through the voltage differential amplifier **A4** (as shown in **Figure 1A**). When the current in the electrodes is 180° out of phase, the electrode current **IE1, IE2** will flow from one electrode to the other, and the resulting differential voltage signal at the output of the differential amplifier **A4** will be proportional to the sum of the electrode-tissue impedances **Z1, Z2,** since the body impedance is negligible. According to an exemplary embodiment, the electrode-tissue impedances of the electrodes **Z1, Z2,** is greater than 1 MΩ. According to another exemplary embodiment, the electrodes **E1, E2** are non-contact electrodes. It is understood that, according to exemplary embodiments, the invention is also advantageous for implementations in which the electrodes **E1, E2** are dry-contact electrodes.

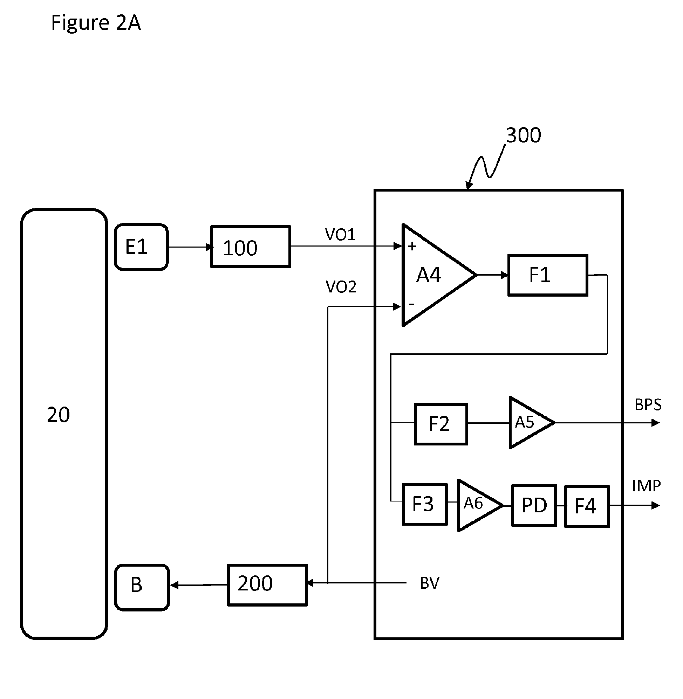

**Figure 2A** shows a second exemplary block diagram of a system for acquisition of biopotential signals according to an embodiment of the invention. The figure illustrates two electrodes **E1, B** which are applied to a human body **20,** and now the second electrode **B** being a bias electrode connected to a biasing circuit **200** and to the signal processing unit **300** that generates the biasing voltage **BV.** It is understood that in other possible exemplary embodiment, the first electrode voltage **VO1** and the second electrode voltage **VO2** can be interchanged at the input of the differential amplifier **A4.**

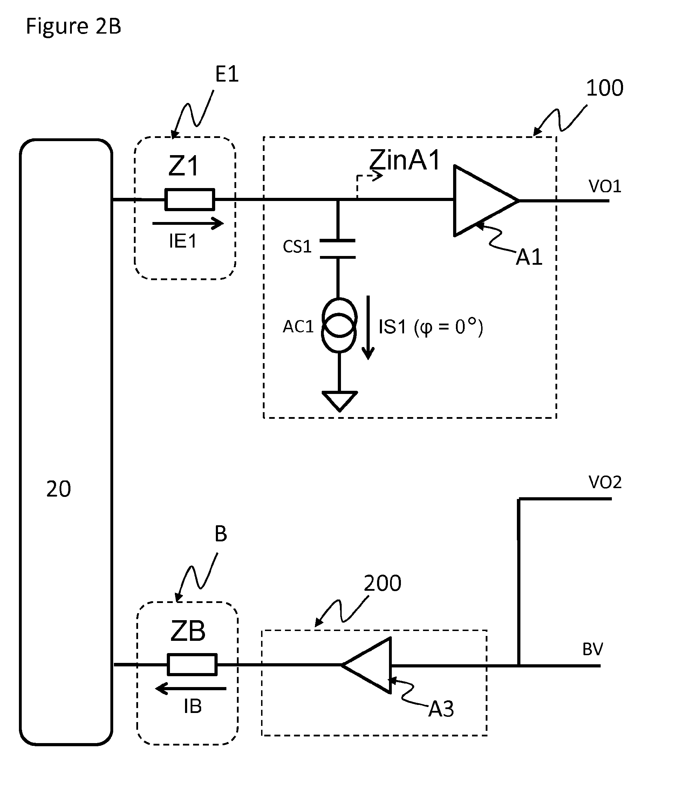

**Figure 2B** shows a more detailed representation of some aspects of the system of **Figure 2A****,** and more specifically, the electrodes **E1, B,** the impedance detection module **100** and the biasing circuit **200.** The electrodes **E1, B** are represented by an electrode-tissue impedance **Z1, ZB** which is connected, in case of the first electrode **E1,** to an impedance detection module **100** as described in **Figure 1B****,** and in case of the second electrode **B,** to the biasing circuit **200** comprising an amplifier **A3.** According to an embodiment of the invention, the bias electrode **B** has a low electrode-tissue impedance **ZB** compared to the first electrode's electrode-tissue impedance **Z1,** e.g. greater than 1 MΩ, and the resulting differential voltage signal at the output of the differential amplifier **A4** will be proportional to the electrode-tissue impedance of the first electrode **Z1,** since the body impedance is negligible. According to another exemplary embodiment, the first electrode **E1** may be a non-contact or a dry-contact electrode.

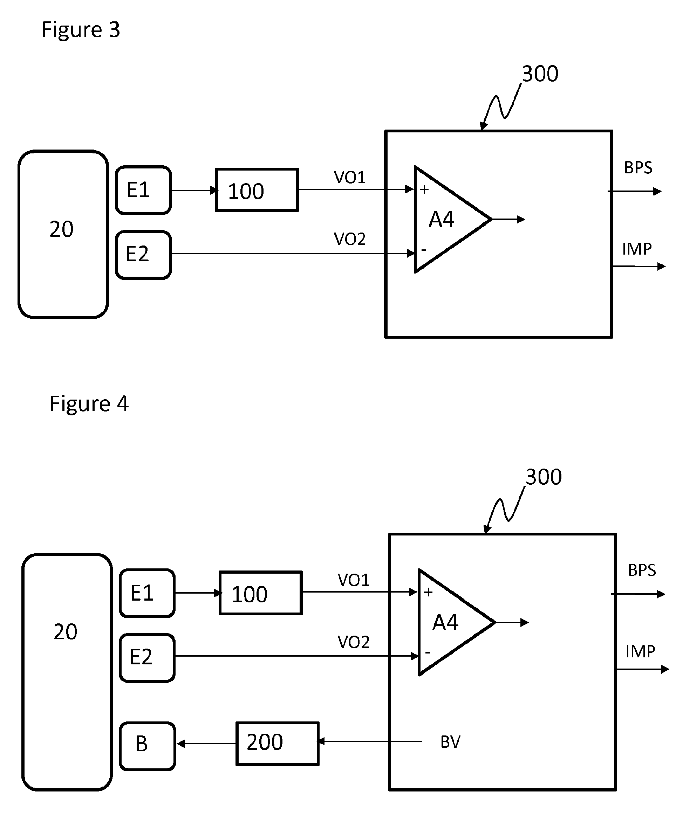

**Figure 3** shows a third exemplary block diagram of a system for acquisition of biopotential signals according to an embodiment of the invention. The figure illustrates two electrodes **E1, E2** which are applied to a human body **20,** and now the second electrode **E2** is a low electrode-tissue impedance electrode, e.g. a contact electrode, which generates a second electrode voltage **VO2.** The first electrode **E1** is connected to the impedance detection module **100** as described in **Figure 1B** and providing a first electrode voltage signal **VO1.** It is also understood that in other possible exemplary embodiment, the first electrode and the second electrode voltage signals **VO1, VO2** can be interchanged at the input of the differential amplifier **A4.** According to an exemplary embodiment, the first electrode **E1** is a non-contact or a dry-contact electrode. According to an embodiment the first electrode **E1** has an electrode-tissue impedance **Z1** greater than 1 MΩ. According to an embodiment of the invention, the second electrode **E2** has low electrode-tissue impedance **Z2** compared to the first electrode's electrode-tissue impedance **Z1** and the resulting differential voltage signal at the output of the differential amplifier **A4** will be proportional to the electrode-tissue impedance of the first electrode **Z1,** since the body impedance is negligible.

**Figure 4** shows a fourth exemplary block diagram of a system for acquisition of biopotential signals according to an embodiment of the invention. The figure illustrates three electrodes **E1, E2, B** which are applied to a human body **20,** and now the second electrode **E2** is a low electrode-tissue impedance electrode, e.g. a contact electrode, which generates a second electrode voltage **VO2,** and the third electrode **B** is a bias electrode connected to a biasing circuit **200,** as disclosed in **Figure 2B****,** and to the signal processing unit **300** that generates the biasing voltage **BV.** The first electrode **E1** is connected to the impedance detection module **100** as described in **Figure 1B** and providing a first electrode voltage signal **VO1.** It is also understood that in other possible exemplary embodiment, the first electrode and the second electrode voltage signals **VO1, VO2** can be interchanged at the input of the differential amplifier **A4.** According to another exemplary embodiment, the first electrode **E1** is a non-contact electrode. According to an exemplary embodiment the first electrode **E1** has an electrode-tissue impedance **Z1** greater than 1 MΩ. According to an exemplary embodiment, this configuration works as the one disclosed in **Figure 3** but with improved rejection of common mode noise or CMRR. According to exemplary embodiments the invention is also advantageous for applications with dry-contact electrodes.

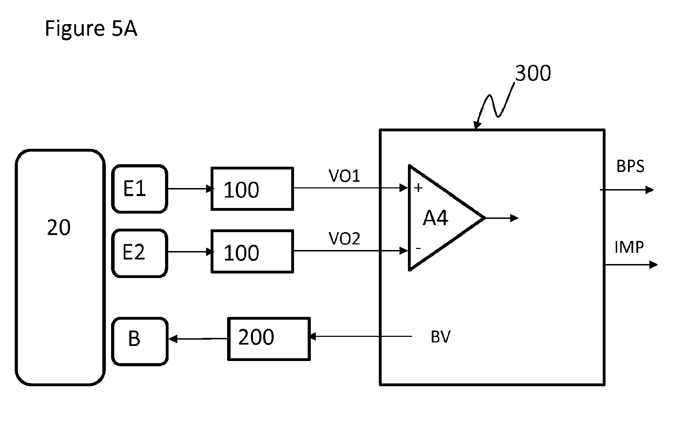

**Figure 5A** shows a fifth exemplary block diagram of a system for acquisition of biopotential signals according to an embodiment of the invention. The figure illustrates three electrodes **E1, E2, B** which are applied to a human body **20,** wherein the first and the second electrodes **E1, E2** are connected to an impedance detection module **100** as described in **Figure 1** **B,** and generate a first and a second electrode voltage **VO1, VO2.** The third electrode **B** is a bias electrode connected to a biasing circuit 200, as described in **Figure 2B****,** and to the signal processing unit **300** that generates the biasing voltage **BV.** According to an exemplary embodiment, the first and second electrodes **E1, E2** are non-contact electrodes. According to an exemplary embodiment the first and second electrodes **E1, E2** have an electrode-tissue impedance **Z1, Z2** greater than 1 MΩ. The bias electrode **B** may be a low electrode-tissue impedance electrode, e.g. a contact electrode. According to exemplary embodiments the invention is also advantageous for applications in which the first and/or the second electrode **E1, E2** is a dry-contact electrode.

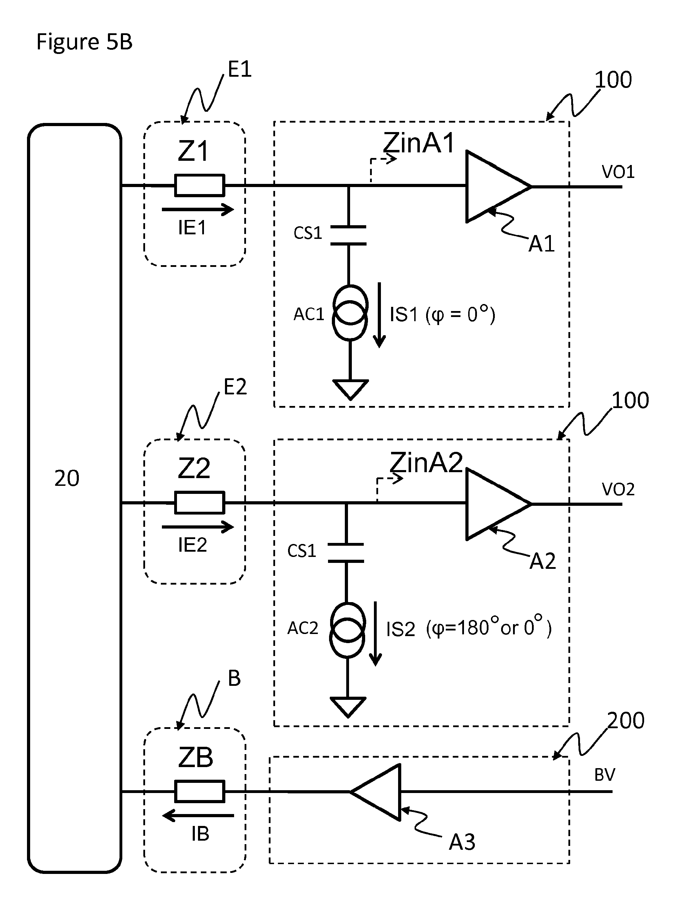

**Figure 5B** shows a more detailed representation of some aspects of the system of **Figure 5A****,** and more specifically, the electrodes **E1, E2, B,** the impedance detection module **100** and the biasing circuit **200.** The electrodes **E1, E2, B** are represented by an electrode-tissue impedance **Z1, Z2, ZB** which is connected, in case of the first and second electrodes **E1, E2,** to an impedance detection module **100** as described in **Figure 1B****,** and in case of the bias electrode **B,** to the biasing circuit **200** comprising an amplifier **A3.**

According to an embodiment of the invention, the impedance detection module **100** applies an AC current **IS1, IS2** of constant amplitude and frequency into the electrodes **E1, E2** and the resulting differential voltage between the electrodes **VO1, VO2** is measured through the voltage differential amplifier **A4** (as shown in **Figure 1A**). When the current in the electrodes is 180° out of phase, the electrode current **IE1, IE2** will flow from one electrode to the other, and the resulting differential voltage signal at the output of the differential amplifier **A4** will be proportional to the sum of the electrode-tissue impedances **Z1, Z2,** since the body impedance is negligible. When the current in both electrodes **IE1, IE2** is in phase, the net resulting current will flow into the bias electrode **B,** and the resulting differential voltage signal at the output of the differential amplifier **A4** will be proportional to the difference of the of the electrode-tissue impedances **Z1, Z2,** since the body impedance is negligible.

It is understood that without parasitics, the current through the electrodes **IE1, IE2** equals the generated AC current **IS1, IS2** in the current generation circuit **40.**

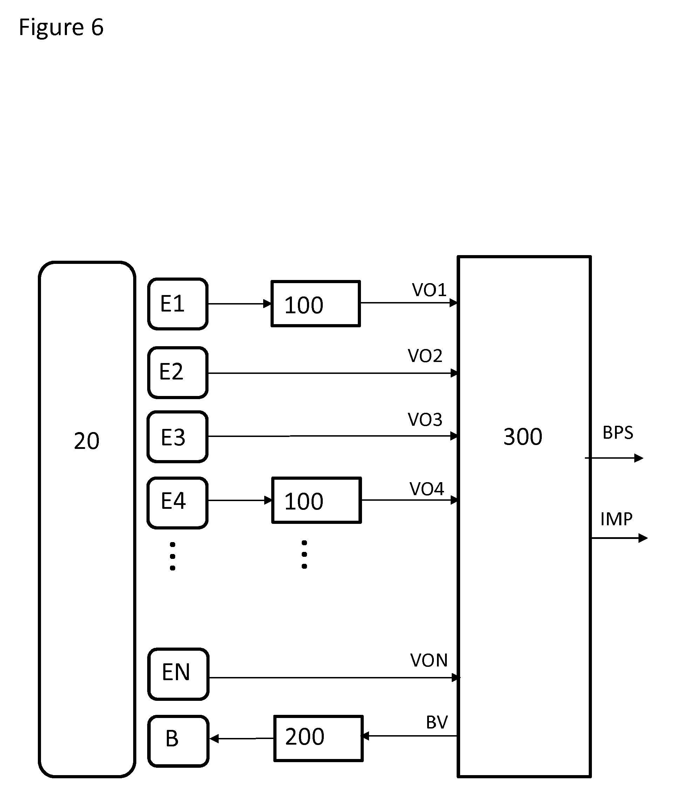

**Figure 6** shows a sixth exemplary block diagram of a system for acquisition of biopotential signals according to an embodiment of the invention. The figure illustrates N+1 electrodes **E1** to **EN, B** which are applied to a human body **20,** wherein some of the electrodes, for example **E1** and **E4,** are connected to an impedance detection module **100** as described in **Figure 1B****,** and some, for example **E2, E3, EN,** are not. A bias electrode **B** is also present, connected to a biasing circuit **200,** as described in **Figure 2B****,** and to the signal processing unit **300** that generates the biasing voltage **BV.** According to an embodiment of the invention, the signal processing unit **300** may consider any pair of electrode voltages **VO1** to **VON, BV** in order to calculate a component value of a first or a first and a second electrode-tissue impedance based on the difference between a first electrode voltage and a second electrode voltage, as is described in previous figures and embodiments. According to exemplary embodiments, the electrodes **E1** and/or **E4** may be non-contact and/or dry-contact electrodes.

## Claims

1. A system for acquisition of biopotential signals, comprising:
at least a first electrode (E1) configured for detecting a biopotential signal within a signal bandwidth of interest and being connected to an impedance detection module (100) which provides a first electrode voltage (VO1);
said impedance detection module (100) comprising a current generation circuit (40) with an AC current generator (AC1) configured to generate a first current signal (IS1) through said first electrode, said first current signal (IS1) having a frequency outside of the signal bandwidth of interest; and an amplifier (A1) connected in parallel to said current generation circuit (40); and
signal processing means (300) for calculating a component value (IMP) of at least a first electrode-tissue impedance (Z1, Z2, ZB) based on the difference between the first electrode voltage (VO1) and a second electrode voltage (VO2 to VON; BV);
**characterized in that**
the current generation circuit (40) comprises a capacitor (CS1) connected between the input of the amplifier (A1) and the AC current generator (AC1) so as to isolate said AC current generator from said amplifier input at the signal bandwidth of interest.

2. A system for acquisition of biopotential signals according to claim 1, wherein the first electrode-tissue impedance (Z1) is greater than 1 megohm.

3. A system for acquisition of biopotential signals according to any of the previous claims, wherein the signal bandwidth of interest is below 250 hertz.

4. A system for acquisition of biopotential signals according to any of the previous claim, wherein the first current signal (IS1) has a frequency greater than 1 kilohertz.

5. A system for acquisition of biopotential signals according to any of the previous claims, wherein the value of the current generation circuit capacitor (CS1) is designed such as to reduce the amplifier's input impedance (ZinA1) by less than 25%.

6. A system for acquisition of biopotential signals according to any of the previous claims, wherein the value of the current generation circuit capacitor (CS1) is designed such as to obtain values of the current generation circuit impedance (Zcg) greater than 10 gigaohm at the signal bandwidth of interest.

7. A system for acquisition of biopotential signals according to any of the previous claims, wherein the value of the current generation circuit capacitor (CS1) is in the range between 0.1 to 20 picofarad.

8. A system for acquisition of biopotential signals according to any of the previous claims, wherein the AC current generator (AC1) is designed so as to have an output impedance which is at least 5 times higher than the impedance of the capacitor (CS1) at the frequency of the first current signal (IS1).

9. A system for acquisition of biopotential signals according to any of the previous claims, wherein the AC current generator (AC1) is implemented as a Howland current pump.

10. A system for acquisition of biopotential signals according to any of the previous claims, wherein the first electrode (E1) is a non-contact or a dry-contact electrode.

11. A system for acquisition of biopotential signals according to any of the previous claims, further comprising:
a second electrode (E2) configured for detecting a biopotential signal within a signal bandwidth of interest and being connected to an impedance detection module (100) which provides a second electrode voltage (VO2); said impedance detection module (100) comprising a current generation circuit (40) with an AC current generator (AC2) configured to generate a second current signal (IS2) through said second electrode; and
wherein the first current signal (IS1) and the second current signal (IS2) are 180 degrees out of phase.

12. A system for acquisition of biopotential signals according to any of claims 1 to 10, further comprising:
a second electrode (E2) configured for detecting a biopotential signal within a signal bandwidth of interest and being connected to an impedance detection module (100) which provides a second electrode voltage (VO2); said impedance detection module (100) comprising a current generation circuit (40) with an AC current generator (AC2) configured to generate a second current signal (IS2) through said second electrode;
a bias electrode (B) configured for biasing the subjects body (20); and
so arranged that when the first current signal (IS1) and the second current signal (IS2) are in phase, the net resulting current flows into the bias electrode.

13. A method for acquisition of biopotential signals, comprising:
detecting a biopotential signal within a signal bandwidth of interest with at least a first electrode (E1);
generating a first current signal (IS1) through said first electrode, said first current signal (IS1) having a frequency outside of the signal bandwidth of interest;
isolating said first current signal from said detected biopotential signal at the signal bandwidth of interest;
generating a first and a second electrode voltage (VO1 to VON; BV); and
calculating a component value (IMP) of at least a first electrode-tissue impedance (Z1, Z2, ZB) based on the difference between the first electrode voltage (VO1) and a second electrode voltage (VO2 to VON; BV).
